# FASCICULE DE BREVET EUROPEEN

(11) **EP 0 764 436 B1**
(45) Date de publication et mention de la délivrance du brevet: **24.02.1999**
(21) Numéro de dépôt: 96401823.8
(22) Date de dépôt: 26.08.1996
(51) Int. Cl.: A61K 7/06, A61K 7/11, A61K 7/043

(54) **Composition cosmétique ou dermatologique aqueuse comprenant un oligomère filmogène et des particules nanométriques rigides et non-filmifiables; utilisations**
Wässrige kosmetische oder dermatologische Zubereitung, enthaltend ein filmbildendes Oligomer und harte und nicht filmbildende Nanopartikel; Verwendung
Aqueous cosmetic or dermatological composition comprising a filmogenic oligomer and rigid, non-filmforming nanoparticles; uses

(30) Priorité: 21.09.1995 FR 9511109; 03.10.1995 FR 9511615
(43) Date de publication de la demande: 26.03.1997
(73) Titulaire: L'OREAL, 75008 Paris (FR)
(72) Inventeur: Mougin, Nathalie, 75011 Paris (FR); Bauer, Daniel, 93340 Le Raincy (FR); Mondet, Jean, 93600 Aulnay-sous-Bois (FR); Samain, Henri, 91570 Bievres (FR); Franbourg, Alain, 75010 Paris (FR)
(74) Mandataire: Miszputen, Laurent

(56) Documents cités:
- EP-A- 0 214 626
- EP-A- 0 288 012
- EP-A- 0 320 218
- EP-A- 0 628 304
- FR-A- 2 091 516
- FR-A- 2 528 699

## Description

La présente invention a pour objet des compositions cosmétiques ou dermatologiques comprenant, dans un milieu aqueux cosmétiquement acceptable, au moins un oligomère filmogène, soluble ou dispersible dans ledit milieu, et au moins des particules nanométriques rigides, non-filmogènes et dispersées dans ledit milieu, ainsi que leurs utilisations dans le domaine de la cosmétique ou de la dermopharmacie, notamment dans les produits pour le maintien de la coiffure, les produits pour le revêtement des cils et des sourcils, les produits pour le maquillage et/ou le soin des ongles.

Les polymères filmogènes solubles dans les milieux aqueux et hydroalcooliques tels que la polyvinylpyrrolidone, les copolymères de vinylpyrrolidone/acétate de vinyle, les copolymères d'acétate de vinyle/acide crotonique, les résines acryliques anioniques ou amphotères sont couramment utilisés dans les produits pour la fixation des cheveux notamment ceux décrits dans la demande de brevet EP-A-0288 012, les mascaras ou les produits pour le soin et/ou le maquillage des ongles.

Les produits capillaires pour la fixation des cheveux les plus répandus sur le marché de la cosmétique sont des compositions à pulvériser essentiellement constituées d'une solution le plus souvent hydroalcoolique et d'un polymère filmogène soluble dans l'eau et dans l'alcool tels que ceux cités ci-dessus, en mélange avec divers adjuvants cosmétiques. Cette solution est généralement conditionnée soit dans un récipient aérosol approprié mis sous pression à l'aide d'un gaz propulseur, soit dans un flacon pompe.

Depuis un certain nombre d'années, un intérêt tout particulier s'est manifesté pour les laques aérosols ou flacons pompes à fortes concentrations d'eau "à haut extrait sec" de polymère filmogène.

Dans toute la description, on entendra par aérosol ou flacon pompe à 〈〈haut extrait sec〉〉 en produit laquant, toute formulation aqueuse conditionnée sous l'une de ces formes contenant plus de 5 % en poids en matière sèche de produit laquant par rapport au poids total de ladite formulation.

D'une part, on cherche à diminuer les concentrations en composés volatils à la pression atmosphérique dits COV (Composé Organique Volatile) présents dans les compositions à pulvériser sous forme d'aérosol ou de flacon pompe. En effet, l'emploi d'alcool, seul ou en mélange avec une faible quantité d'eau ainsi que l'utilisation de gaz propulseurs peut présenter certains inconvénients tels que l'augmentation de l'inflammabilité ou la nuisance vis-à-vis de l'environnement.

Les COV sont principalement les gaz propulseurs tels que les hydrocarbures ou le diméthyléther (DME) et les solvants tels que l'éthanol.

D'autre part, on cherche à réduire les temps de séchage du produit pulvérisé et à augmenter son pouvoir laquant après pulvérisation sur les cheveux. L'utilisation des polymères filmogènes hydrosolubles dans les aérosols ou flacons pompes à fortes concentrations d'eau, en particulier dans les systèmes pulvérisateurs eau/éthanol/ diméthyléther ayant un taux de COV maximal de 55 %, nécessite des concentrations plus élevées en extrait sec de polymère par rapport à celles utilisées dans les systèmes pulvérisateurs organiques (100 % COV) pour obtenir des pouvoirs de fixation et des temps de séchage satisfaisants. En effet, l'augmentation de la concentration en eau dans les conditionnements aérosols ou flacons pompes conduit à une forte diminution du pouvoir laquant ainsi qu'à des temps de séchage beaucoup plus longs.

Les polymères filmogènes habituellement utilisés dans ce type de compositions ont des poids moléculaires mesurés en chromatographie d'exclusion stérique en général supérieurs à 50.000 et de préférence supérieurs à 100.000. L'augmentation de leur concentration dans des laques aérosols ou flacons pompes à fortes teneurs en eau, conduit à des viscosités trop élevées de sorte qu'on ne puisse plus obtenir une pulvérisation satisfaisante du produit à la sortie de l'aérosol ou du flacon pompe.

Pour remédier à ces problèmes de viscosité du 〈〈jus〉〉 dans le dispositif de pulvérisation, une solution consisterait à utiliser des oligomères filmogènes solubles ou dispersibles dans le milieu de la composition à pulvériser, apportant une faible viscosité. Or, la plupart des oligomères, de poids moléculaire inférieur à 50.000, utilisés dans les produits pour le maintien des cheveux présentent des propriétés mécaniques insuffisantes pour obtenir un pouvoir laquant satisfaisant, même à des concentrations élevées.

La demanderesse a découvert de manière surprenante qu'en associant à des oligomères filmogènes, de-poids moléculaire inférieur à 50.000, solubles ou dispersibles dans les milieux aqueux, des particules nanométriques, rigides et non-filmogènes, on pouvait réaliser des laques aérosols ou des flacons pompes à haut extrait sec en agent laquant, ayant une bonne diffusion lors de l'application, un bon pouvoir laquant et une bonne vitesse de séchage. Cette association est tout particulièrement adaptée aux compositions capillaires aérosols à 〈〈haut extrait sec〉〉 du type eau/éthanol/DME ayant un taux de COV maximal de 55 %.

La demanderesse a découvert que cette association particulière de polymères constituait un agent de revêtement des matières kératiniques, ayant à la fois de bonnes propriétés cosmétiques notamment au niveau du toucher et du démêlage, de bonnes propriétés filmogènes, des propriétés mécaniques satisfaisantes pour la fixation des cheveux, de bonnes propriétés d'adhésion sur les cils et les sourcils ainsi que des propriétés mécaniques satisfaisantes pour le revêtement des ongles.

Après application sur le support kératinique et séchage, on obtient le dépôt d'un matériau composite dont la structure est constituée d'une matière continue hydrophile formée par l'oligomère filmifié, ladite matrice contenant une multitude de nodules rigides constitués par les particules nanométriques non-filmogènes.

Selon les proportions respectives de l'oligomère filmogène et des particules non-filmifiables et rigides, on peut réaliser des compositions cosmétiques capillaires présentant un bon pouvoir laquant, une bonne résistance à l'humidité en réduisant l'effet de poissage par hydroscopicité que pourrait apporter l'oligomère hydrophile et une bonne élimination au lavage en raison du caractère hydrophile de la matrice continue du dépôt composite.

L'association conforme à la présente invention permet également la réalisation de produits pour le revêtement des cils et des sourcils en particulier de mascaras crèmes et de mascaras résistants à l'eau (〈〈waterproof〉〉), présentant un bon pouvoir d'adhésion sur les cils et les sourcils, sans effet collant en surface du film et facilement éliminables au lavage avec un démaquillant à faible teneur en tensioactif.

L'association conforme à l'invention permet également la réalisation de produits pour le maquillage et/ou le soin des ongles présentant une bonne adhérence sur l'ongle, une bonne dureté et une bonne rigidité. Ils sont de plus totalement éliminables à l'eau ou en présence d'un démaquillant à faible teneur en tensioactif.

Les compositions cosmétiques ou dermatologiques, conformes à l'invention sont caractérisées par le fait qu'elles contiennent dans un milieu aqueux cosmétiquement acceptable au moins :
(A) un oligomère filmogène soluble ou dispersible dans ledit milieu, ayant un poids moléculaire mesuré par chromatographie d'exclusion stérique, inférieur ou égal à 50.000 ;
(B) des particules rigides et non-filmogènes dont la taille moyenne est inférieure ou égale à 1 µm, dispersées dans ledit milieu.

Les oligomères solubles ou dispersibles dans le milieu aqueux des compositions de l'invention ont de préférence un poids moléculaire mesuré en chromatographie d'exclusion stérique allant de 500 à 45.000.

Leur température T'g de transition vitreuse varie, de préférence, de - 50°C à + 50° C et plus particulièrement de - 30 à + 40° C. Ils sont préférentiellement solubles dans les milieux aqueux ou hydroalcooliques.

Les oligomères utilisés dans les compositions selon l'invention peuvent être non-ioniques tels que les copolymères de vinylpyrrolidone/acétate de vinyle. Ils peuvent être amphotères comme les copolymères N-octylacrylamide/ méthacrylate de méthyle/ méthacrylate d'hydroxypropyle/acide acrylique/ méthacrylate t-butylaminoéthyle tels que le produit vendu sous le nom AMPHOMER par la Société National Starch. Ils peuvent être également anioniques.

Les oligomères particulièrement préférés sont les oligomères anioniques contenant des monomères porteurs de groupes anioniques ionisés ou ionisables en particulier des groupes acide carboxylique et/ou des groupes acide sulfonique dans des proportions inférieures ou égales à 30% en poids par rapport au poids de l'oligomère. Ces groupes sont de préférence partiellement ou totalement neutralisés pour obtenir une meilleure solubilité de l'oligomère dans le milieu aqueux de la composition et une meilleure élimination au lavage.

Parmi les oligomères anioniques utilisés selon l'invention, on peut citer les copolymères d'esters vinyliques tels que l'acétate de vinyle, le propionate de vinyle, le néodécanoate de vinyle, les benzoates de vinyle substitués ou non, avec un monomère du type acide maléique ou acide crotonique. On peut citer en particulier le copolymère d'acétate de vinyle/acide crotonique de composition identique à celle du produit LUVISET CA66 vendu par la Société BASF, mais de poids moléculaire inférieur ou égal à 50.000.

On peut citer également les copolymères d'esters d'acide (méth)acrylique en C₁-C₈ comme par exemple l'acrylate de tertio-butyle, l'acrylate d'éthyle, le méthacrylate de méthyle, l'acrylate d'isobutyle avec le monomère acide acrylique et/ou le monomère acide méthacrylique. On peut utiliser par exemple un copolymère acrylate d'éthyle/acrylate de tertio-butyle/acide méthacrylique de composition identique à celle du produit vendu sous le nom LUVIMER 100 P par BASF, mais de poids moléculaire inférieur ou égal à 50.000.

Les oligomères filmogènes selon l'invention peuvent être préparés de façon classique par polymérisation ou copolymérisation radicalaire en solution, suspension ou en émulsion.

Ils sont préparés de préférence en solution dans un solvant organique à partir du ou des monomères en mélange dans le solvant en présence d'un initiateur de radicaux libres. Ils peuvent être ensuite purifés par précipitation dans un solvant tel que l'éther de pétrole.

On peut également opérer en semi-continu en utilisant un pied de cuve ne contenant que la partie solvant, une faible partie du mélange de monomères et une partie de l'amorceur. On chauffe ensuite à la température de réaction et on effectue une double coulée simultanée du reste du mélange des monomères et du reste d'amorceur dissous dans une quantité de solvant.

Dans le cas des oligomères anioniques, les oligomères ainsi obtenus peuvent être ensuite partiellement ou totalement neutralisés par un composé monobasique non-volatil telle qu'une base minérale comme la soude ou la potasse, ou un aminoalcool par exemple pris dans le groupe constitué par l'amino-2 méthyl-2 propanol-1 (AMP), la triéthanolamine, la triisopropanolamine (TIPA), la monoéthanolamine, la tri[(hydroxy-2) propyl-1 amine, l'amino-2 méthyl-2 propanediol-1,3 (AMPD), l'amino-2 hydroxyméthyl-2 propanediol-1,3.

La taille moyenne des particules rigides et non-filmogènes de l'invention est inférieure à 1 µm, de préférence inférieure à 500 nm, plus préférentiellement inférieure à 300 nm et plus particulièrement inférieure à 100 nm.

Les particules rigides et non-filmogènes, utilisées selon la présente invention sont de préférence des particules de polymère ayant une température de transition vitreuse supérieure à 50° C et plus particulièrement supérieure à 70° C.

La polydispersité en taille des particules de polymère dispersées dans le mileu des compositions de l'invention, mesurée en diffusion quasi élastique de lumière, est de préférence inférieure à 0,35.

Les particules de polymère rigides et non-filmogènes de l'invention sont plus préférentiellement constituées de polymère réticulé.

Les agents réticulants sont choisis de préférence parmi ceux couramment utilisés en polymérisation radicalaire. On peut citer par exemple les diacrylates ou diméthacrylates d'éthylèneglycol, de polyéthylèneglycol, de propylèneglycol, du divinylbenzène, du di-ou tri-méthacrylate de pentaérythritol ; les diacrylates ou diméthylacrylates d'alkylènediols comme le diméthacrylate d'hexanediol. Ils sont utilisés à des quantités allant préférentiellement de 0,1 à 50 % en poids par rapport au poids des monomères constituant le polymère du latex.

Dans les milieux aqueux contenant des composés organiques volatils, en particulier ceux présents dans les laques aérosols ou les flacons pompes de l'invention, on utilise de préférence une dispersion de particules de polymère comportant des groupes anioniques ionisés ou ionisables, en particulier des groupes acide carboxylique ou acide sulfonique pour apporter une bonne stabilisation du latex (en particulier dans un milieu hydroalcoolique).

Ces groupes acides sont, de préférence présents dans des quantités inférieures ou égales à 10 % en poids, plus particulièrement inférieures ou égales à 8 % en poids et encore plus particulièrement allant de 3 à 8 % en poids par rapport au poids du polymère.

Ces groupes acides sont de préférence partiellement ou totalement neutralisés par une base minérale volatile ou un aminoalcool tels que définis ci-dessus.

Parmi les polymères constituant les particules rigides et non-filmifiables de l'invention, on peut citer par exemple les polymères ou copolymères, de préférence réticulés, obtenus par polymérisation ou copolymérisation d'un monomère ou d'un mélange de monomères choisis dans le groupe constitué par les acrylates ou les méthacrylates d'alkyle linéaire, cyclique ou ramifié en C₁-C₁₀ tels que le méthylméthacrylate de méthyle, le méthacrylate de tertio-butyle, le cyclohexyméthacrylate, l'acrylate ou le méthacrylate d'isobornyle ; le styrène ; le vinyltoluène ; le chlorure de vinyle, le benzoate de vinyle ou le tertiobutylbenzoate de vinyle ; l'acide acrylique, l'acide méthacrylique.

Les polymères particulièrement préférés sont les copolymères réticulés d'au moins un méthacrylate d'alkyle linéaire, cyclique ou ramifié en C₁-C₈ et d'acide acrylique et/ou d'acide méthacrylique.

Les dispersions de particules de polymère ou "latex" selon l'invention peuvent être obtenues par polymérisation en émulsion en batch, selon un procédé comprenant :
a) la réalisation d'un pied de cuve dans le réacteur contenant de l'eau, éventuellement un tampon et un agent émulsionnant ;
b) l'addition dans le pied de cuve, à température ambiante, des monomères ;
c) la mise en émulsion des monomères ;
d) le chauffage à la température de polymérisation du milieu réactionnel en présence d'un amorceur radicalaire.

On peut également opérer en semi-continu en utilisant un pied de cuve ne contenant que la partie aqueuse, une faible partie du mélange de monomères et une partie de l'amorceur. On chauffe ensuite à la température de réaction et on effectue une double coulée simultanée du reste du mélange des monomères et du reste d'amorceur dissous dans une quantité d'eau.

Le milieu aqueux cosmétiquement acceptable de l'invention, est constitué de préférence par de l'eau ou un mélange d'eau et d'au moins un solvant cosmétiquement acceptable et compatible avec le latex rigide et non-filmogène et l'oligomère filmogène tel qu'un monoalcool, un polyalcool, un éther de glycol, l'acétone ou un ester, seul ou sous forme de mélange. Il est plus particulièrement constitué d'eau ou d'eau et d'un alcool inférieur en C₁-C₄ comme l'éthanol ou l'isopropanol.

La concentration en solvant organique dans composition de l'invention est de préférence comprise entre 15 et 35 % en poids et plus particulièrement entre 20 et 30 % en poids par rapport au poids total de la composition.

Lorsque la composition selon l'invention est conditionnée sous pression dans un dispositif aérosol en vue d'obtenir une laque, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges ; le gaz carbonique, le protoxyde d'azote, le diméthyléther, l'azote ou l'air comprimé. La concentration du gaz propulseur dans le dispositif aérosol dépend de la nature du propulseur choisi.

Le diméthyléther est particulièrement préféré. Il est utilisé dans les laques aérosols de l'invention comme gaz propulseur dans des concentrations allant de préférence de 30 à 45% en poids par rapport au poids total de la composition.

La concentration en composé organique volatil (COV) dans une composition selon l'invention conditionnée sous forme d' aérosol ou de flacon pompe est de préférence inférieure ou égale à 55% en poids et plus particulièrement comprise entre 30 et 55% en poids par rapport au poids total de la formulation conditionnée en aérosol ou en flacon pompe.

Le pH des compositions selon l'invention est généralement compris entre 2 et 9 et en particulier entre 3 et 8. Il peut être ajusté à la valeur choisie au moyen d'agents alcanisants ou acidifiants habituellement utilisés en cosmétique.

Dans le mélange constitué par les particules rigides et non-filmogènes et par l'oligomère filmogène, les proportions en particules varient de préférence, de 5 à 95 % en poids en matière sèche par rapport au poids du mélange. Ces proportions varient en fonction de l'application envisagée de la composition.

Lorsque les compositions de l'invention se présentent sous forme de laque aérosol ou de flacon pompe, les proportions en matière sèche des particules rigides et non-filmogènes varient, de préférence, de 5 à 60 % en poids et celles en oligomère varient de 40 à 95% en poids par rapport au poids du mélange particules/oligomère. Le matériau composite obtenu après dépôt et séchage sur support kératinique est constituée d'une matrice continue d'oligomère filmifié contenant une multitude de particules nanométriques rigides qui servent à augmenter la rigidité de la matrice.

Lorsque les compositions de l'invention se présentent sous forme de laque aérosol ou de flacon pompe à haut extrait sec, il est également possible d'utiliser un mélange constitué d'une forte concentration en matière sèche de particules rigides et non-filmogènes, allant de préférence de 70 à 95 % en poids par rapport au poids du mélange particules non-filmogènes/oligomère. Ce mode particulier de réalisation de l'invention permet d'obtenir au dépôt un matériau composite principalement constitué d'une matrice de particules rigides collées entre elles par l'oligomère filmogène, ayant un bon pouvoir laquant sur les cheveux. Ce mode de réalisation permet d'optimiser la viscosité du jus dans le dispositif de pulvérisation en utilisant des concentrations plus faibles en oligomère tout en obtenant un bon pouvoir laquant.

Les compositions selon l'invention peuvent contenir éventuellement en plus un agent plastifiant pour améliorer les propriétés mécaniques, les propriétés cosmétiques et l'adhésion sur les matières kératiniques du matériau composite déposé après application et séchage. La présence d'un agent plastifiant n'est pas obligatoire pour ajuster le pouvoir laquant dans les formulations laques de l'invention contrairement aux formulations laques classiques.

Parmi les agents plastifiants pouvant être utilisés selon l'invention, on peut citer :
- les CARBITOLS de la Société UNION CARBIDE à savoir le CARBITOL ou diéthylène glycol éthyléther, le méthyl CARBITOL ou diéthylène glycol méthyléther, le butyl CARBITOL ou diéthylène glycol butyléther ou encore l'hexyl CARBITOL ou diéthylène glycol hexyléther,
- les CELLOSOLVES de la Société UNION CARBIDE à savoir le CELLOSOLVE ou éthylène glycol éthyléther, le butyl CELLOSOLVE ou éthylène glycol butyléther, l'hexyl CELLOSOLVE ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les DOWANOLS de la Société DOW CHEMICAL à savoir le DOWANOL PM ou propylène glycolméthyléther, le DOWANOL DPM ou dipropylène glycol méthyléther et le DOWANOL TPM ou tripropylène glycol méthyléther.

On peut encore citer :
- le diéthylène glycol méthyléther ou DOWANOL DM de la Société DOW CHEMICAL,
- l'huile de ricin oxyéthylénée à 40 moles d'oxyde d'éthylène telle que celle vendue par la Société RHÔNE POULENC sous la dénomination de "MULGOFEN LE-719",
- l'alcool benzylique,
- le citrate de triéthyle vendu par la Société PFIZER sous la dénomination de "CITROFLEX-2",
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2 hexyle, et
- les esters de glycérol tels que le diacétate de glycérol (diacétine) et le triacétate de glycérol (triacétine).

L'agent plastifiant est présent en une proportion allant, préférentiellement, de 0 à 20% en poids par rapport au poids du mélange constitué par l'oligomère filmogène et les particules non filmifiables. Cette proportion varie selon l'application envisagée.

Un objet de l'invention conceme l'utilisation de l'association constituée de la dispersion aqueuse de particules rigides et non filmogènes et de l'oligomère filmogène telle que définie ci-dessus comme agent de revêtement des matières kératiniques dans et pour la préparation 'd'une composition cosmétique ou dermatologique.

Les compositions selon l'invention telles que définies ci-dessus peuvent être utilisées comme base de produit capillaire pour la mise en forme et/ou le maintien de la coiffure en particulier des laques aérosol ou des flacons pompes pour fixer les cheveux, des lotions pour la mise en plis ou le brushing, les mousses de coiffage.

Les compositions capillaires pour le maintien de la coiffure conformes à la présente invention contiennent, de préférence, le mélange constitué par les particules rigides et non-filmogènes et par l'oligomère filmogène dans des proportions allant de 3 à 20 % en poids en matière sèche par rapport au poids total de la composition.

Les compositions capillaires conformes à l'invention peuvent contenir en plus des additifs cosmétiques conventionnels tels que des conservateurs, des adoucissants, des séquestrants, des parfums, des colorants, des modificateurs de viscosité, des agents nacrants, des agents hydratants, des agents antipelliculaires, des agents anti-séborrhéiques, des filtres solaires, des agents de conditionnement pour les cheveux, des anti-oxydants, des protéines, des vitamines.

Les compositions selon la présente invention peuvent être utilisées comme base de produit aqueux de revêtement des cils et des sourcils tel qu'un mascara notamment un mascara «crème» ou un mascara résistant à l'eau (waterproof).

Les compositions pour le revêtement des cils et des sourcils conformes à l'invention contiennent l'association de l'invention telle que définie précédemment dans des proportions allant, préférentiellement de 2 à 15 % en poids en matière sèche par rapport au poids total de la composition. Le milieu aqueux cosmétiquement acceptable est généralement de l'eau ou un milieu hydroalcoolique.

Les compositions pour le revêtement des cils et des sourcils conformes à l'invention comportent en général au moins une cire.

Les cires utilisées dans les compositions de mascara selon l'invention sont choisies parmi les cires, solides et rigides, d'origine animale, végétale, minérale ou de synthèse et leurs mélanges.

Parmi les cires animales, on peut citer la cire d'abeilles, la cire de lanoline, et les cires d'insectes de Chine.

Parmi les cires végétales, on peut notamment citer la cire de riz, la cire de carnauba, la cire de candellila, la cire d'ouricurry, la cire de fibres de liège, la cire de canne à sucre, la cire du Japon et la cire de sumac.

Parmi les cires minérales, on peut notamment citer la cire de montan, les cires microcristallines, les paraffines et l'ozokérite.

Parmi les cires de synthèse, on peut notamment citer les cires de polyéthylène, les cires obtenues par la synthèse de FISHER et TROPSCH et les copolymères cireux ainsi que leurs esters.

On peut également utiliser dans les compositions selon l'invention, les cires obtenues par hydrogénation catalytique d'huiles animales ou végétales ayant des chaînes grasses, linéaires, ou ramifiées, en C₈-C₃₂.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.

Les compositions de mascara selon l'invention peuvent en outre contenir des pigments.

Ces pigments peuvent être organiques ou minéraux ou peuvent être également des pigments nacrés. De tels pigments sont bien connus et sont en particulier décrits dans FR 83-09997 (2.528.699).

Les compositions de mascara selon l'invention peuvent se présenter sous différentes formes. Elles peuvent en particulier se présenter sous forme d'émulsions huile-dans-eau ou eau-dans-huile ou sous forme de dispersions.

Les compositions mascaras peuvent contenir également des agents tensio-actifs anioniques et/ou des agents tensio-actifs non ioniques.

Parmi les agents tensio-actifs anioniques pouvant être utilisés seuls ou en mélange, on peut citer notamment les sels alcalins, les sels d'ammonium, les sels d'amine ou les sels d'amino-alcool des composés suivants :
- les alcoylsulfates, alcoyléther sulfates, alcoylamide sulfates, éther sulfates, alcoylarylpolyéther sulfates, monoglycéride sulfates,
- les alcoylsulfonates, alcoylamide sulfonates, alcoylarylsulfonates, α-oléfines sulfonates, paraffines sulfonates,
- les acoylsulfosuccinates, alcoyléther sulfosuccinates, les alcoylamides sulfosuccinates,
- les alcoylasulfosuccinamates,
- les alcoylsulfoacétates, alcoylpolyglycérol carboxylates,
- les alcoylphosphates/alcoyléther phosphates,
- les alcoylsarcosinates, alcoylpolypeptidates, alcoylamidopolypeptidates, les alcoylisothionates, et alcoyltaurates.

On entend par le terme alcoyle utilisé ci-dessus une chaîne hydrocarbonée ayant généralement de 12 à 18 atomes de carbone.

On peut citer également comme agents tensio-actifs anioniques utilisables dans les compositions selon l'invention des sels d'acides gras, tels que ceux de l'acide oléique, ricinoléique, palmitique, stéarique, les acides d'huile de coprah ou d'huile de coprah hydrogénée, et en particulier les sels d'amines tels que les stéarates d'amines.

On peut encore citer comme agents tensio-actifs anioniques les acyl lactylates dont le radical acyle comprend de 8 à 20 atomes de carbone, et les acides carboxyliques d'éthers polyglycoliques répondant à la formule :

R₄-(OCH₂-CH₂)ₙ-OCH₂-COOH

dans laquelle :
R₄ représente un radical alkyle linéaire ayant de 12 à 18 atomes de carbone et n est un nombre entier compris entre 5 et 15, et les sels desdits acides. On utilise de préférence comme agent tensio-actif anionique des stéarates d'amines.

Parmi les agents tensio-actifs non ioniques pouvant être utilisés seuls ou en mélange dans les compositions de mascara selon l'invention, on peut citer notamment les alcools, les alcoylphénols et les acides gras polyéthoxylés, polypropoxylés ou polyglycérolés à chaîne grasse ayant de 8 à 18 atomes de carbone.

On peut également citer des copolymères d'oxydes d'éthylène et de propylène, des condensats d'oxydes d'éthylène et de propylène sur des alcools gras, des amides gras polyéthoxylés, des amines grasse polyéthoxylées, des éthanolamides, des esters d'acides gras de glycol, des esters d'acides gras du sorbitan oxyéthylénés ou non, des esters d'acides gras du saccharose, des esters d'acides gras des polyéthylèneglycols, des triesters phosphoriques et des esters d'acides gras de dérivés du glucose.

On peut également citer les produits de condensation d'un monoalcool, d'un alpha-diol, d'un alcoylphénol, d'un amide ou d'un diglycolamide avec le glycidol ou un précurseur du glycidol tel que décrit dans le brevet FR 71-17206 (2.091.516), de formule :

R₅-CHOH-CH₂-O-(CH₂-CHOH-CH₂-O)ₚ-H

dans laquelle :
R₅ représente un radical aliphatique, cycloaliphatique, cycloaliphatique, ou arylaliphatique ayant de préférence entre 7 et 21 atomes de carbone, les chaînes aliphatiques pouvant comporter des groupements éther, thioéther ou hydroxyméthylène et p est un nombre entier compris entre 1 et 10.

On peut en outre, citer les composés décrits dans le brevet FR 1.477.048 de formule :

R₆O-[C₂H₃O-(CH₂OH)]_{q}-H

dans laquelle :
R₅ représente un radical alcoyle, alcényle ou alcoyaryle, et q est une valeur statistique comprise entre 1 et 10.

On peut encore citer les composés décrits dans le brevet français FR 76-31975 (2.328.763) de formule :

R₇CONH-CH₂-CH₂O-CH₂-CH₂O-(CH₂-CHOH-CH₂-O)ᵣ-H

dans laquelle ;
R₇ représente un radical aliphatique linéaire ou ramifié, saturé ou insaturé, pouvant comporter éventuellement un ou plusieurs groupement(s) hydroxyle(s), ayant entre 8 et 30 atomes de carbone, d'origine naturelle ou synthétique, et r est un nombre entier ou décimal compris entre 1 et 5 et désigne le degré de condensation moyen.

Les compositions de mascara selon l'invention peuvent comprendre en outre au moins un additif conventionnel choisi parmi un adoucissant, un conservateur, un agent séquestrant, un parfum, un agent épaississant, une huile, une silicone, un agent de cohésion, un agent alcalinisant ou acidifiant et une charge.

Les agents épaississants utilisables dans les compositions de mascara selon l'invention peuvent être d'origine naturelle ou synthétique.

Parmi les agents épaississants d'origine synthétique, on peut citer notamment les dérivés cellulosiques hydrosolubles, les dérivés d'éthers de cellulose possédant des groupes ammonium quaternaires, les dérivés de l'amidon, les polysaccharides cationiques, les sels de polymères acryliques ou méthacryliques, les polyènes et les polysiloxanes. On peut également obtenir un épaississement des compositions de mascara selon l'invention par adjonction d'un mélange de polyéthylène glycol et de stéarate et/ou de distéarate de polyéthylène glycol ou d'un mélange d'esters phosphoriques et d'amides gras.

Parmi les charges pouvant être utilisées dans les compositions de mascara selon l'invention, on peut citer notamment celles décrites dans FR 91-10791 (2.680.681).

Les compositions pour le revêtement des cils conformes à l'invention présentent une bonne adhérence sur le cil, sans effet collant en surface du film et s'éliminent très facilement à l'aide d'un démaquillant aqueux contenant une faible quantité de tensio-actifs.

Les compositions selon l'invention peuvent également être utilisées comme bases de produits aqueux de revêtement des ongles facilement et complètement éliminables par simple lavage des mains à l'eau, sans avoir à utiliser de démaquillant à forte concentration en tensio-actif ni même d'eau chaude et/ou d'eau fortement savonneuse.

Elles peuvent être utilisées comme sous-couche d'un vernis à ongles à solvant classique, comme vernis aqueux à élimination facile ou bien comme base de soin des ongles contenant en solution ou en dispersion dans le milieu aqueux de la composition des actifs tels que des agents protecteurs, durcisseurs et/ou pour le soin des ongles.

Lorsque ladite composition est utilisée comme sous-couche de vernis solvant, on constate que l'ongle n'a pas tendance à se teinter lors de l'application ultérieure du vernis à solvant, comme cela est le cas dans l'état de la technique ; ceci étant dû au fait que l'ongle n'est pas en contact direct avec ledit vernis.

Lorsque l'on introduit des pigments dans lesdites compositions, elles peuvent être utilisées comme vernis.

Dans le mode particulier d'utilisation comme soin des ongles, on peut imaginer de déposer la base contenant les agents actifs sur l'ongle le soir, ce qui permet le traitement de l'ongle durant la nuit, puis une élimination de cette base le matin par simple lavage des mains.

Parmi les agents actifs utilisables, on peut citer les vitamines et leurs dérivés ; les matières premières d'origine biologique et leurs dérivés telles que la kératine, les protéines, les hydrolysats, le chitosane, la mélanine, les oligo-éléments, le collagène ; la glycérine ; les phospholipides ; l'urée ; le formal.

Les compositions pour le revêtement de l'ongle selon l'invention contiennent l'association de particules rigides et non-filmogènes et de l'oligomère filmogène dans des proportions allant, préférentiellement, de 15 à 30 % en poids en matière sèche par rapport au poids total de la composition.

Un autre objet de l'invention consiste en un procédé de traitement non-thérapeutique des matières kératiniques telles que les cheveux, les cils ou les ongles, caractérisée en ce qu'il consiste à appliquer directement sur les matières kératiniques une composition cosmétique telle que définie précédemment.

Les exemples qui suivent servent à illustrer l'invention sans toutefois présenter un caractère limitatif.

### EXEMPLES

### EXEMPLE1 : Préparation d'un latex acrylique non-filmogène compatible dans un milieu laque aérosol eau/éthanol/DME à taux de COV maximal de 55%

Composition du polymère du latex :

| | |
|---|---|
| - méthacrylate de méthyle | 91 % en poids |
| - acide méthacrylique | 5 % en poids |
| - diméthacrylate d'éthylène glycol (réticulant) | 4 % en poids |

### Mode opératoire :

Dans un réacteur cylindrique avec agitation centrale mécanique, thermomètre, réfrigérant et barbotage d'azote on introduit le mélange suivant :

| | |
|---|---|
| - eau permutée | 28 g |
| - NaHCO₃ | 0,7 g |
| - nonylphénolpolyoxyéthylène (à 25 motifs oxyéthylène) et sulfaté en bout de chaîne vendu sous le nom AD 33 par la société SEPPIC | 6,56 g |

On dissout sous agitation et barbotage d'azote. Puis on ajoute, sous agitation et à température ambiante, le mélange de monomères constitué par:

| | |
|---|---|
| - méthacrylate de méthyle | 91 g |
| - acide méthacrylique | 5 g |
| - diméthylacrylate d'éthylène glycol(réticulant) | 4 g |

L'émulsion des monomères est réalisée à 250 tours/minutes avec barbotage d'azote. On porte cette émulsion à 72° C et on ajoute, à cette température, l'amorceur c'est-à-dire : 0,5 g de persulfate de potassium dissous dans 20 g d'eau permutée.

On laisse réagir dans ces conditions à 72° C durant 3 heures puis on élève la température à 85° C et on laisse à nouveau réagir pendant 5 heures.

On revient ensuite à température ambiante sous agitation. On récupère le latex et on filtre.

On obtient un latex stable de concentration en matière sèche de polymère égale à 25 % en poids.

La température Tg mesurée par DSC (Calorimétrie Différentielle) est de 115 °C.

On détermine les tailles de particules par diffusion quasi élastique de lumière sur un appareil COULTER N4 SD de la Société COULTRONIX:

| | |
|---|---|
| - taille moyenne des particules | 65 nm |
| - polydispersité en taille | < 0,1 |

### EXEMPLE 2 : Préparation d'un oligomère acrylique filmogène compatible dans un milieu laque aérosol eau/éthanol/DME à taux de COV maximal de 55%

Composition de l'oligomère :

| | |
|---|---|
| - acrylate d'isobutyle | 50% en poids |
| - acrylate de tertio-butyle | 40% en poids |
| - acide acrylique | 10% en poids |

### Mode opératoire :

Dans un réacteur cylindrique avec agitation centrale mécanique, thermomètre, réfrigérant et barbotage d'azote on introduit le mélange suivant :

| | |
|---|---|
| - acrylate d'isobutyle | 50 g |
| - acrylate de tertio-butyle | 40 g |
| - acide méthacrylique | 10 g |
| - azobisisobutylacrylonitrile (amorceur) | 2 g |
| - éthanol | 200 g |

On porte sous agitation et barbotage d'azote au reflux de l'éthanol (78 °C). On laisse réagir 12 heures dans ces conditions. On revient à température ambiante. On purifie ensuite le polymère par précipitation de la solution alcoolique dans 5 l d'éther de pétrole. On sèche ensuite le précipité jusqu'à obtention d'un poids constant.

Le rendement obtenu après séchage est de 90%. L'indice d'acide obtenu est 81,5. Le poids moléculaire en sommet de pic mesuré en chromatographie d'exclusion stérique est de 36800 (élution dans le tétrahydrofurane par rapport à des étalons polystyrène). La température T'g mesurée par DSC est de 27 °C.

On réalise une solution alcoolique concentré d'oligomère neutralisé à 100% par l'amino-2 méthyl-2 propanol-1 (AMP) à partir du mélange suivant :

| | |
|---|---|
| - Oligomère de l'exemple 2 | 100 g |
| - AMP | 12,44 g |
| - Ethanol | 112,44 g |

On agite pendant 24 heures à température ambiante. On obtient ainsi une solution à 50% en poids en matière sèche d'oligomère.

### EXEMPLE 3 : Préparation d'un oligomère acrylique filmogène compatible dans un milieu laque aérosol eau/éthanol/DME à taux de COV maximal de 55%

Composition de l'oligomère :

| | |
|---|---|
| - acrylate d'isobutyle | 90% en poids |
| - acide acrylique | 10% en poids |

### Mode opératoire :

Dans un réacteur cylindrique avec agitation centrale mécanique, thermomètre, réfrigérant et barbotage d'azote on introduit le mélange suivant :

| | |
|---|---|
| - acrylate d'isobutyle | 90 g |
| - acide acrylique | 10 g |
| - azobisisobutylacrylonitrile | 2 g |
| - éthanol | 200 g |

On opère dans les mêmes conditions que celles de l'exemple 2.

Le rendement obtenu après séchage est de 95%. L'indice d'acide obtenu est 81. Le poids moléculaire en sommet de pic mesuré en chromatographie d'exclusion stérique est de 33400 (élution dans le tétrahydrofurane par rapport à des étalons polystyrène). La température T'g mesurée par DSC est de 0 °C.

On réalise une solution alcoolique concentré d'oligomère neutralisé à 100% par l'amino-2 méthyl-2 propanol (AMP) à partir du mélange suivant :

| | |
|---|---|
| - Oligomère de l'exemple 3 | 100 g |
| - AMP | 12,44 g |
| - Ethanol | 112,44 g |

On agite pendant 24 heures à température ambiante. On obtient ainsi une solution d'oligomère à 50% de matière sèche.

### EXEMPLE 4 : Préparation d'un oligomère filmogène compatible dans un milieu laque aérosol eau/éthanol/DME à taux de COV maximal de 55%

Composition de l'oligomère :

| | |
|---|---|
| - acide crotonique | 10% en poids |
| - acétate de vinyle | 90% en poids |

### Mode opératoire :

Dans un réacteur cylindrique avec agitation centrale mécanique, thermomètre, réfrigérant et barbotage d'azote on introduit successivement pour constituer le 〈〈pied de cuve〉〉 le mélange suivant:

| | |
|---|---|
| - acide crotonique | 1 g |
| - acétate de vinyle | 9 g |
| - acétate d'éthyle | 80 g |
| - tertio-butyl peroxy2-éthylhexanoate vendu sous le nom TRIGONOX 21 S par la société AKZO | 0,7 g |

On porte sous agitation et barbotage à 78 °C (reflux) et on laisse réagir pendant 15 minutes. On commence alors la double coulée simultanément du reste du mélange de monomères d'une part et de l'amorceur dissous dans le reste de solvant d'autre part. Pour cela, on a introduit dans une première ampoule à introduction le mélange de monomères constitué par:

| | |
|---|---|
| - acide crotonique | 9 g |
| - acétate de vinyle | 81 g |

Ce mélange est coulé dans le milieu réactionnel sous agitation et à reflux pendant 4 heures. Simultanément, on a introduit dans une deuxième ampoule à introduction le mélange constitué de :

| | |
|---|---|
| - acétate d'éthyle | 80 g |
| - tertio-butyl peroxy2-éthylhexanoate vendu sous le nom TRIGONOX 21 S par la société AKZO | 2 g |

Ce deuxième mélange est coulé simultanément au premier dans le milieu réactionnel pendant 4 h 30.

A la fin des deux additions, on laisse réagir à nouveau 2 heures au reflux. On procède alors à la coulée finale du reste d'amorceur dans le solvant. Pour cela, on a introduit à nouveau dans une ampoule à introduction le mélange constitué de :

| | |
|---|---|
| - acétate d'éthyle | 30 g |
| - tertio-butyl peroxy2-éthylhexanoate vendu sous le nom TRIGONOX 21 S par la société AKZO | 1 g |

Ce mélange est additionné en 1 heure dans le milieu sous agitation et au reflux.

On laisse réagir à nouveau 4 heures à la fin de l'introduction. On ramène ensuite à température ambiante et on purifie le polymère en précipitant la solution dans 10 l d'éther de pétrole. Le précipité est séché jusqu'à obtention d'un poids constant.

| | |
|---|---|
| Rendement: | 76,2 % |
| Indice d'acide : (soit 11,7% d'acide crotonique) | 75,9 % |
| Température T'g mesurée par DSC : | 40 °C |
| Poids moléculaire mesurée en chromatographie d'exclusion stérique : | 23.000 |

### EXEMPLE 5 : Laque aérosol eau/éthanol/DME à 55% de COV pour la fixation des cheveux contenant un mélange constitué de 30%en poids en matière sèche de latex rigide et non-filmogène et 70%en poids en matière sèche d'oligomère.

### COMPOSITION AVANT CONDITIONNEMENT :

| | |
|---|---|
| - Solution alcoolique à 50% en poids d'oligomère de l'exemple 2 | 166,70g |
| - Eau permutée | 854,87 g |
| - Ethanol | 395,72 g |
| - Dispersion à 25% en poids de particules non filmogènes de l'exemple 1 | 100,00g |

### LAQUE AEROSOL A 55% EN COV :

| | |
|---|---|
| - Composition ci-dessus | 35 g |
| - Diméthyléther | 15 g |

Cette laque contient 5% en poids de matière sèche.

Après application sur les cheveux, on obtient un excellent pouvoir laquant, de bonnes propriétés cosmétiques et une bonne élimination du dépôt au shampooing.

### EXEMPLE 6 : Laque aérosol eau/éthanol/DME à 55% de COV pour la fixation des cheveux contenant un mélange constitué de 50% en poids en matière sèche de latex rigide et non-filmogène et 50%en poids en matière sèche d'oligomère.

### COMPOSITION AVANT CONDITIONNEMENT :

| | |
|---|---|
| - Solution alcoolique à 50% en poids d'oligomère de l'exemple 2 | 50,00 g |
| - Eau permutée | 354,18 g |
| - Ethanol | 196,10 g |
| - Dispersion à 25% en poids de particules non filmogènes de l'exemple 1 | 100,00 g |

### LAQUE AEROSOL A 55% EN COV :

| | |
|---|---|
| - Composition ci-dessus | 35 g |
| - Diméthyléther | 15 g |

Cette laque contient 5% en poids de matière sèche

Après application sur les cheveux, on obtient un excellent pouvoir laquant, de bonnes propriétés cosmétiques et une élimination totale du dépôt au shampooing.

### EXEMPLE 7 : Laque aérosol eau/éthanol/DME à 55% de COV pour la fixation des cheveux contenant un mélange constitué de 50%en poids en matière sèche de latex rigide et non-filmogène et 50%en poids en matière sèche d'oligomère

### COMPOSITION AVANT CONDITIONNEMENT:

| | |
|---|---|
| - Solution alcoolique à 50% en poids d'oligomère de l'exemple 3 | 50,00 g |
| - Eau permutée | 354,18 g |
| - Ethanol | 196,10 g |
| - Dispersion à 25% en poids de particules non filmogènes de l'exemple 1 | 100,00 g |

### LAQUE AEROSOL A 55% EN COV:

| | |
|---|---|
| - Composition ci-dessus | 35 g |
| - Diméthyléther | 15 g |

Cette laque contient 5% en poids de matière sèche.

Après application sur les cheveux, on obtient un excellent pouvoir laquant, de bonnes propriétés cosmétiques et une élimination totale du dépôt au shampooing.

### EXEMPLE 8 : Laque aérosol eau/éthanol/DME à 55% de COV pour la fixation des cheveux contenant un mélange constitué de 50%en poids en matière sèche de latex rigide et non-filmogène et 50%en poids en matière sèche d'oligomère.

### COMPOSITION AVANT CONDITIONNEMENT :

| | |
|---|---|
| - Oligomère de l'exemple 4 | 3,20 g |
| - AMP pour neutraliser à 100% l'oligomère | 0,38 g |
| - Eau permutée | 50,58 g |
| - Ethanol | 31,57 g |
| - Dispersion à 25% en poids de particules non filmogènes de l'exemple 1 | 14,28 g |

### LAQUE AEROSOL A 55% EN COV :

| | |
|---|---|
| - Composition ci-dessus | 35 g |
| - Diméthyléther | 15 g |

Cette laque contient 5% en poids de matière sèche.

Après application sur les cheveux, on obtient un excellent pouvoir laquant, de bonnes propriétés cosmétiques et une élimination totale du dépôt au shampooing.

### EXEMPLE 9 : Laque aérosol eau/éthanol/DME pour la fixation des cheveux à 45% de COV contenant un mélange constitué de 50%en poids en matière sèche de latex rigide et non-filmogène et 50% en poids en matière sèche d'oligomère filmogène

### COMPOSITION AVANT CONDITIONNEMENT :

| | |
|---|---|
| - Solution alcoolique à 50% en poids d'oligomère de l'exemple 2 | 11,54 g |
| - Eau permutée | 55,06 g |
| - Ethanol | 10,32 g |
| - Dispersion à 25% en poids de particules non filmogènes de l'exemple 1 | 23,08 g |

### LAQUE AEROSOL A 45% EN COV :

| | |
|---|---|
| - Composition ci-dessus | 65 g |
| - Diméthyléther | 35 g |

Cette laque contient 7,5% en poids de matière sèche.

Après application sur les cheveux, on obtient un excellent pouvoir laquant, de bonnes propriétés cosmétiques et une élimination totale du dépôt au shampooing.

### EXEMPLE 10 : Laque aérosol eau/éthanol/DME pour la fixation des cheveux à 33,5% de COV contenant un mélange constitué de 75% en poids en matière sèche de latex rigide et non-filmogène et 25% en poids en matière sèche d'oligomère filmogène

### COMPOSITION AVANT CONDITIONNEMENT :

| | |
|---|---|
| - Solution alcoolique à 50% en poids d'oligomère de l'exemple 2 | 10 g |
| - Eau permutée | 30 g |
| - Dispersion à 25% en poids de particules non filmogènes de l'exemple 1 | 60 g |

### LAQUE AEROSOL A 33,5% EN COV :

| | |
|---|---|
| - Composition ci-dessus | 70 g |
| - Diméthyléther | 30 g |

Cette laque contient14% en poids de matière sèche.

Après application sur les cheveux, on obtient un très faible remouillage des cheveux malgré la forte quantité d'eau contenue dans le spray, une fixation rapide de la coiffure et un bon pouvoir laquant, un toucher agréable et un démêlage facile au brossage. On observe également une élimination totale du dépôt au shampooing.

## Revendications

1. Composition cosmétique ou dermatologique, caractérisée par le fait qu'elle contient dans un milieu aqueux cosmétiquement acceptable au moins :
(A) un oligomère filmogène soluble ou dispersible dans ledit milieu, ayant un poids moléculaire mesuré par chromatographie d'exclusion stérique, inférieur ou égal à 50.000 ;
(B) des particules rigides et non-filmogènes de polymère ayant une température de transition vitreuse Tg supérieure à 50 °C dont la taille moyenne est inférieure ou égale à 1 µm, dispersées dans ledit milieu.

2. Composition selon la revendication 1, caractérisée par le fait que l'oligomère filmogène a un poids moléculaire mesuré en chromatographie d'exclusion stérique allant de 500 à 45.000.

3. Composition selon la revendication 1 ou 2, caractérisée par le fait que l'oligomère filmogène a une température de transition vitreuse T'g allant de - 50 °C à + 50 °C.

4. Composition selon l'une quelconque des revendications 1 à 3, selon laquelle l'oligomère filmogène est soluble dans les milieu aqueux ou hydroalcoliques.

5. Composition selon l'une quelconque des revendications 1 à 4, selon laquelle l'oligomère filmogène est un polymère non-ionique, anionique ou amphotère.

6. Composition selon l'une quelconque des revendications 1 à 5, selon laquelle l'oligomère filmogène est un polymère anionique portant des groupes anioniques ionisés ou ionisables pouvant être partiellement ou totalement neutralisés.

7. Composition selon la revendication 6, selon laquelle les groupes anioniques sont des groupes acide carboxylique et/ou acide sulfonique

8. Composition selon la revendication 7, selon laquelle les groupes acide carboxylique et/ou acide sulfonique sont présent dans des proportions inférieures ou égales à 30% en poids par rapport au poids de l'oligomère.

9. Composition selon l'une quelconque des revendications 1 à 8, selon laquelle l'oligomère filmogène est choisi dans le groupe constitué par les copolymères d'esters vinyliques et d'acide crotonique et/ou d'acide maléïque : les copolymères d'esters d'acide (méth)acrylique en C₁-C₈ linéaires, ramifiés ou cycliques et d'acide acrylique et/ou d'acide méthacrylique.

10. Composition selon l'une quelconque des revendications 1 à 9, selon laquelle les particules rigides et non-filmogènes ont une taille moyenne inférieure ou égale à 500 nm et de préférence inférieure ou égale à 300 nm et plus particulièrement inférieure ou égale à 100 nm.

11. Composition selon l'une quelconque des revendications 1 à 10, selon laquelle les particules rigides et non-filmogènes de polymère ont une température de transition vitreuse Tg supérieure à 70 °C.

12. Composition selon la revendication 11, selon laquelle la polydispersité en taille des particules de polymère rigides et non-filmogènes, mesurée en diffusion quasi élastique de lumière, est inférieure à 0,35.

13. Composition selon l'une quelconque des revendications 11 et 12, selon laquelle le polymère constituant les particules rigides et non-filmogènes est réticulée.

14. Composition selon la revendication 13, caractérisée par le fait que l'agent réticulant est présent dans des proportions allant de 0,1 à 50% en poids par rapport au poids dudit polymère.

15. Composition selon l'une quelconque des revendications 11 à 14, selon laquelle le polymère constituant les particules rigides et non-filmogènes est un polymère anionique comportant des groupes anioniques ionisés ou ionisables pouvant être partiellement ou totalement neutralisés.

16. Composition selon la revendication 15, selon laquelle les groupes anioniques sont des groupes acide carboxylique et/ou acide sulfonique.

17. Composition selon la revendication 16, selon laquelle les groupes acide carboxylique et/ou acide sulfonique sont présents dans des proportions inférieures ou égales à 10% en poids par rapport au poids du polymère.

18. Composition selon l'une quelconque des revendications 11 à 17, caractérisée par le fait que le polymère constituant les particules rigides et non-filmifiables est un polymère ou copolymère obtenu par polymérisation ou copolymérisation d'un monomère ou un mélange de monomères choisis dans le groupe constitué par les acrylates ou méthacrylates d'alkyle linéaire, cyclique ou ramifié en C₁-C₁₀, le styrène, le vinyltoluène, le chlorure de vinyle, le benzoate de vinyle, le tertiobutylbenzoate de vinyle, l'acide acrylique, l'acide méthacrylique.

19. Composition selon l'une quelconque des revendications 11 à 18, caractérisée par le fait que que le polymère constituant les particules rigides et non-filmifiables est un copolymère réticulé d'au moins un méthacrylate d'alkyle linéaire, cyclique ou ramifié en C₁-C₈ et d'acide acrylique et/ou d'acide méthacrylique.

20. Composition selon l'une quelconque des revendications 1 à 19, caractérisée par le fait que le milieu aqueux cosmétiquement acceptable est constitué par de l'eau ou un mélange d'eau et d'au moins un solvant cosmétiquement acceptable et compatible avec les particules rigides et non-filmogènes et l'oligomère filmogène.

21. Composition selon la revendication 20, caractérisée par le fait que le solvant est choisi dans le groupe constitué par des monoalcools, des polyalcools, des éthers de glycol ou des esters d'acide gras, seuls ou en mélange.

22. Composition selon l'une quelconque des revendications 1 à 21, caractérisée par le fait que le milieu aqueux cosmétiquement acceptable est constitué d'eau ou d'eau et d'un alcool inférieur en C₁-C₄.

23. Composition selon l'une quelconque des revendications 1 à 22, caractérisée par le fait que la concentration en solvant organique est comprise entre 15 et 35 % en poids par rapport au poids total de la composition.

24. Composition selon l'une quelconque des revendications 1 à 23, caractérisée par le fait qu'elle est conditionnée dans un dispositif aérosol sous pression ou un flacon pompe.

25. Composition selon la revendication 24, caractérisée par le fait qu'elle est conditionnée dans un dispositif aérosol sous pression en présence d'un agent propulseur.

26. Composition selon la revendication 25, selon laquelle l'agent propulseur est choisi dans le groupe constitué par les hydrocarbures volatils, les hydrocarbures chlorés et/ou fluorés et leurs mélanges ; le gaz carbonique, le protoxyde d'azote, le diméthyléther, l'azote ou l'air comprimé.

27. Composition selon la revendication 25, selon laquelle l'agent propulseur est le diméthyléther.

28. Composition selon l'une quelconque des revendications 25 à 27, selon laquelle la concentration en diméthyléther est comprise entre 30 et 55% en poids par rapport au poids total de la composition.

29. Composition selon l'une quelconque des revendications 24 à 28, selon laquelle la concentration en composé organique volatil (COV) est inférieure ou égale à 55% en poids par rapport au poids total de la composition conditionnée en aérosol ou en flacon pompe.

30. Composition selon l'une quelconque des revendications 1 à 29, caractérisée par le fait que dans le mélange constitué par les particules rigides et non-filmogènes et par l'oligomère filmogène, les proportions en particules varient de 5 à 95 % en poids en matière sèche par rapport au poids du mélange.

31. Composition selon l'une quelconque des revendications 24 à 30, caractérisée par le fait que dans le mélange constitué par les particules rigides et non-filmogènes et par l'oligomère filmogène, les proportions en particules varient de 40 à 95 % en poids en matière sèche par rapport au poids du mélange.

32. Composition selon l'une quelconque des revendications 24 à 30, caractérisée par le fait que dans le mélange constitué par les particules rigides et non-filmogènes et par l'oligomère filmogène, les proportions en particules varient de 70 à 95 % en poids en matière sèche par rapport au poids du mélange.

33. Composition selon l'une quelconque des revendications 1 à 32, caractérisée par le fait qu'elle contient en plus un agent plastifiant.

34. Utilisation de l'association constituée des particules rigides et non filmogènes et de l'oligomère filmogène telle que définie dans l'une quelconque des revendications 1 à 33 comme agent de revêtement des matières kératiniques dans et pour la préparation d'une composition cosmétique ou pour la préparation d'une composition dermatologique.

35. Produit capillaire pour la mise en forme et/ou le maintien de la coiffure, caractérisé par le fait qu'il est constitué par une composition telle que définie selon l'une quelconque des revendications 1 à 33.

36. Produit capillaire selon la revendication 35, caractérisé par le fait qu'il contient le mélange constitué par les particules rigides et non-filmogènes et par l'oligomère filmogène dans des proportions allant de 3 à 20 % en poids en matière sèche par rapport au poids total de la composition.

37. Produit capillaire selon la revendication 35 ou 36, caractérisé par le fait qu'il s'agit d'un produit choisi dans le groupe consitué par les laques aérosol ou les flacons pompes pour fixer les cheveux, les lotions pour la mise en plis et les lotions pour le brushing, les mousses de coiffage.

38. Produit pour le revêtement des cils et des sourcils, caractérisé par le fait qu'il est constitué par une composition telle que définie selon l'une quelconque des revendications 1 à 23, 30 et 33.

39. Produit pour le revêtement des cils et des sourcils selon la revendication 38, caractérisé par le fait qu'il contient le mélange constitué par les particules rigides et non-filmogènes et par l'oligomère filmogène dans des proportions allant de 2 à 15 % en poids en matière sèche par rapport au poids total de la composition.

40. Produit pour le revêtement des cils et des sourcils selon la revendication 38 ou 39, caractérisé par le fait qu'il s'agit d'un mascara..

41. Produit pour le revêtement des ongles, caractérisé par le fait qu'il est constitué par une composition telle que définie selon l'une quelconque des revendications 1 à 23, 30 et 33.

42. Produit pour le revêtement des ongles selon la revendication 41, caractérisé par le fait qu'il contient le mélange constitué par les particules rigides et non-filmogènes et par l'oligomère filmogène dans des proportions allant de 15 à 30% en poids en matière sèche par rapport au poids total de la composition.

43. Produit pour le revêtement des ongles selon la revendication 41 ou 42, caractérisé par le fait qu'il est choisi dans le groupe constitué par un vernis à ongle à élimination facile au lavage, une sous-couche d'un vernis à ongle, une base de soin des ongles contenant des agents protecteurs, durcisseurs et/ou pour le soin des ongles.

44. Procédé de traitement cosmétique des matières kératiniques telles que les cheveux, les cils, les sourcils ou les ongles, caractérisé en ce qu'il consiste à appliquer directement sur les matières kératiniques une composition cosmétique telle que définie selon l'une quelconque des revendications 1 à 33.

## Claims

1. Cosmetic or dermatological composition, characterized in that it contains, in a cosmetically acceptable aqueous medium, at least:
(A) a film-forming oligomer that is soluble or dispersible in the said medium, having a molecular weight, measured by steric exclusion chromatography, of less than or equal to 50,000;
(B) rigid, non-film-forming particles of polymer having a glass transition temperature Tg of higher than 50°C, whose average size is less than or equal to 1 µm, dispersed in the said medium.

2. Composition according to Claim 1, characterized in that the film-forming oligomer has a molecular weight, measured by steric exclusion chromatography, within the range from 500 to 45,000.

3. Composition according to Claim 1 or 2, characterized in that the film-forming oligomer has a glass transition temperature T'g ranging from -50°C to +50°C.

4. Composition according to any one of Claims 1 to 3, according to which the film-forming oligomer is soluble in aqueous or aqueous-alcoholic media.

5. Composition according to any one of Claims 1 to 4, according to which the film-forming oligomer is a nonionic, anionic or amphoteric polymer.

6. Composition according to any one of Claims 1 to 5, according to which the film-forming oligomer is an anionic polymer bearing ionized or ionizable anionic groups which may be partially or totally neutralized.

7. Composition according to Claim 6, according to which the anionic groups are carboxylic acid and/or sulphonic acid groups.

8. Composition according to Claim 7, according to which the carboxylic acid and/or sulphonic acid groups are present in proportions less than or equal to 30% by weight relative to the weight of the oligomer.

9. Composition according to any one of Claims 1 to 8, according to which the film-forming oligomer is chosen from the group consisting of copolymers of vinyl esters and of crotonic acid and/or maleic acid; copolymers of C₁-C₈ linear, branched or cyclic (meth) acrylic acid esters and of acrylic acid and/or methacrylic acid.

10. Composition according to any one of Claims 1 to 9, according to which the rigid, non-film-forming particles have an average size of less than or equal to 500 nm and preferably of less than or equal to 300 nm and more particularly of less than or equal to 100 nm.

11. Composition according to any one of Claims 1 to 10, according to which the rigid, non-film-forming polymer particles have a glass transition temperature Tg of higher than 70°C.

12. Composition according to Claim 11, according to which the size polydispersity of the rigid, non-film-forming polymer particles, measured by quasi-elastic light scattering, is less than 0.35.

13. Composition according to either of Claims 11 and 12, according to which the polymer constituting the rigid, non-film-forming particles is crosslinked.

14. Composition according to Claim 13, characterized in that the crosslinking agent is present in proportions ranging from 0.1 to 50% by weight relative to the weight of the said polymer.

15. Composition according to any one of Claims 11 to 14, according to which the polymer constituting the rigid, non-film-forming particles is an anionic polymer containing ionized or ionizable anionic groups which may be partially or totally neutralized.

16. Composition according to Claim 15, according to which the anionic groups are carboxylic acid and/or sulphonic acid groups.

17. Composition according to Claim 16, according to which the carboxylic acid and/or sulphonic acid groups are present in proportions of less than or equal to 10% by weight relative to the weight of the polymer.

18. Composition according to any one of Claims 11 to 17, characterized in that the polymer constituting the rigid, non-filmifiable particles is a polymer or copolymer obtained by polymerization or copolymerization of a monomer or of a mixture of monomers chosen from the group consisting of C₁-C₁₀ linear, cyclic or branched alkyl acrylates or methacrylates, styrene, vinyltoluene, vinyl chloride, vinyl benzoate, vinyl tert-butylbenzoate, acrylic acid and methacrylic acid.

19. Composition according to any one of Claims 11 to 18, characterized in that the polymer constituting the rigid, non-filmifiable particles is a crosslinked copolymer of at least one C₁-C₈ linear, cyclic or branched alkyl methacrylate and of acrylic acid and/or methacrylic acid.

20. Composition according to any one of Claims 1 to 19, characterized in that the cosmetically acceptable aqueous medium consists of water or a mixture of water and at least one cosmetically acceptable solvent which is compatible with the rigid, non-film-forming particles and the film-forming oligomer.

21. Composition according to Claim 20, characterized in that the solvent is chosen from the group consisting of monoalcohols, polyalcohols, glycol ethers or fatty acid esters, alone or as a mixture.

22. Composition according to any one of Claims 1 to 21, characterized in that the cosmetically acceptable aqueous medium consists of water or of water and a C₁-C₄ lower alcohol.

23. Composition according to any one of Claims 1 to 22, characterized in that the concentration of organic solvent is between 15 and 35% by weight relative to the total weight of the composition.

24. Composition according to any one of Claims 1 to 23, characterized in that it is packaged in an aerosol device under pressure or a pump-dispenser.

25. Composition according to Claim 24, characterized in that it is packaged in an aerosol device under pressure in the presence of a propellant.

26. Composition according to Claim 25, according to which the propellant is chosen from the group consisting of volatile hydrocarbons, chloro- and/or fluorohydrocarbons and mixtures thereof; carbon dioxide, nitrous oxide, dimethyl ether, nitrogen or compressed air.

27. Composition according to Claim 25, according to which the propellant is dimethyl ether.

28. Composition according to any one of Claims 25 to 27, according to which the dimethyl ether concentration is between 30 and 55% by weight relative to the total weight of the composition.

29. Composition according to any one of Claims 24 to 28, according to which the concentration of volatile organic compound (VOC) is less than or equal to 55% by weight relative to the total weight of the composition packaged as an aerosol or as a pump-dispenser.

30. Composition according to any one of Claims 1 to 29, characterized in that in the mixture consisting of the rigid, non-film-forming particles and the film-forming oligomer, the proportions of particles range from 5 to 95% solids by weight relative to the weight of the mixture.

31. Composition according to any one of Claims 24 to 30, characterized in that in the mixture consisting of the rigid, non-film-forming particles and the film-forming oligomer, the proportions of particles range from 40 to 95% solids by weight relative to the weight of the mixture.

32. Composition according to any one of Claims 24 to 30, characterized in that in the mixture consisting of the rigid, non-film-forming particles and the film-forming oligomer, the proportions of particles vary from 70 to 95% solids by weight relative to the weight of the mixture.

33. Composition according to any one of Claims 1 to 32, characterized in that it also contains a plasticizer.

34. Use of the combination consisting of rigid, non-film-forming particles and the film-forming oligomer as defined in any one of Claims 1 to 33, as an agent for coating keratin substances, in and for the preparation of a cosmetic composition or for the preparation of a dermatological composition.

35. Hair product for shaping and/or maintaining the hairstyle, characterized in that it consists of a composition as defined according to any one of Claims 1 to 33.

36. Hair product according to Claim 35, characterized in that it contains the mixture consisting of the rigid, non-film-forming particles and the film-forming oligomer in proportions ranging from 3 to 20% solids by weight relative to the total weight of the composition.

37. Hair product according to Claim 35 or 36, characterized in that it is a product chosen from the group consisting of aerosol lacquers or pump-dispensers for fixing the hair, hairsetting lotions and blow-drying lotions, and styling mousses.

38. Product for coating the eyelashes and the eyebrows, characterized in that it consists of a composition as defined according to any one of Claims 1 to 23, 30 and 33.

39. Product for coating the eyelashes and the eyebrows according to Claim 38, characterized in that it contains the mixture consisting of the rigid, non-film-forming particles and the film-forming oligomer in proportions ranging from 2 to 15% solids by weight relative to the total weight of the composition.

40. Product for coating the eyelashes and the eyebrows according to Claim 38 or 39, characterized in that it is a mascara.

41. Product for coating the nails, characterized in that it consists of a composition as defined according to any one of Claims 1 to 23, 30 and 33.

42. Product for coating the nails according to Claim 41, characterized in that it contains the mixture consisting of the rigid, non-film-forming particles and the film-forming oligomer in proportions ranging from 15 to 30% solids by weight relative to the total weight of the composition.

43. Product for coating the nails according to Claim 41 or 42, characterized in that it is chosen from the group consisting of a nail varnish which is easily removed by washing, a nail varnish undercoat, and a nail care base containing protecting agents and/or hardeners to care for the nails.

44. Process for the cosmetic treatment of keratin substances such as the hair, the eyelashes, the eyebrows or the nails, characterized in that it consists in applying a cosmetic composition as defined according to any one of Claims 1 to 33 directly onto the keratin substances.

## Patentansprüche

1. Kosmetische oder dermatologische Zusammensetzung,
**dadurch gekennzeichnet, daß**
sie in einem kosmetisch akzeptablen Medium mindestens enthält:
(A) ein in dem Medium lösliches oder dispergierbares filmbildendes Oligomer mit einem durch räumliche Ausschlußchromatographie bestimmten Molekulargewicht von höchstens 50.000, und
(B) starre und nicht filmbildende Polymerpartikel mit einer Glasübergangstemperatur T_{g} über 50 °C, deren mittlere Größe höchstens 1 µm beträgt und die in dem Medium dispergiert sind.

2. Zusammensetzung nach Anspruch 1, dadurch gekennzeichnet, daß das filmbildende Oligomer ein mit räumlicher Ausschlußchromatographie bestimmtes Molekulargewicht im Bereich von 500 bis 45.000 aufweist.

3. Zusammensetzung nach Anspruch 1 oder 2, dadurch gekennzeichnet, daß das filmbildende Oligomer eine Glasübergangstemperatur T_{g} im Bereich von - 50 °C bis + 50 °C aufweist.

4. Zusammensetzung nach einem der Ansprüche 1 bis 3, wobei das filmbildende Oligomer in dem wässerigen oder wässerig-alkoholischen Medium löslich ist.

5. Zusammensetzung nach einem der Ansprüche 1 bis 4, wobei das filmbildende Oligomer ein nichtionisches, anionisches oder amphoteres Polymer ist.

6. Zusammensetzung nach einem der Ansprüche 1 bis 5, wobei das filmbildende Oligomer ein anionisches Polymer mit ionisierten oder ionisierbaren anionischen Gruppen ist, welche teilweise oder vollständig neutralisiert sein können.

7. Zusammensetzung nach Anspruch 6, wobei die anionischen Gruppen Carboxygruppen und/oder Sulfonsäuregruppen sind.

8. Zusammensetzung nach Anspruch 7, wobei die Carboxygruppen und/oder Sulfonsäuregruppen in Anteilen von höchstens 30 Gew.-%, bezogen auf das Gewicht des Oligomers, vorliegen.

9. Zusammensetzung nach einem der Ansprüche 1 bis 8, wobei das filmbildende Oligomer unter den Copolymeren von Vinylestern und Crotonsäure und/oder Maleinsäure und den Copolymeren von geradkettigen, verzweigten oder cyclischen (Meth)acrylsäureestern mit 1 bis 8 Kohlenstoffatomen und Acrylsäure und/oder Methacrylsäure ausgewählt sind.

10. Zusammensetzung nach einem der Ansprüche 1 bis 9, wobei die starren und nicht filmbildenden Partikel eine mittlere Größe von höchstens 500 nm und vorzugsweise von höchstens 300 nm und insbesondere von höchstens 100 nm aufweisen.

11. Zusammensetzung nach einem der Ansprüche 1 bis 10, wobei die starren und filmbildenden Polymerpartikel eine Glasübergangstemperatur über 70 °C aufweisen.

12. Zusammensetzung nach Anspruch 11, wobei die Größenpolydispersität der starren und nicht filmbildenden Polymerpartikel, bestimmt mit quasielastsicher Lichtstreuung, unter 0,35 liegt.

13. Zusammensetzung nach einem der Ansprüche 1 bis 11, wobei das Polymer, das die starren und nicht filmbildenden Partikel bildet, vernetzt ist.

14. Zusammensetzung nach Anspruch 13, dadurch gekennzeichnet, daß das Vernetzungsmittel in Anteilen im Bereich von 0,1 bis 50 Gew.-%, bezogen auf das Gewicht das Polymers, vorliegt.

15. Zusammensetzung nach einem der Ansprüche 11 bis 14, wobei das Polymer, das die starren und nicht filmbildenden Partikel bildet, ein anionisches Polymer ist, das ionisierte oder ionisierbare anionische Gruppen trägt, die teilweise oder vollständig neutralisiert sein können.

16. Zusammensetzung nach Anspruch 17, wobei die anionischen Gruppen Carbonsäuregruppen und/oder Sulfonsäuregruppen sind.

17. Zusammensetzung nach Anspruch 16, wobei die Carboxy- und/oder Sulfonsäuregruppen in Mengenanteilen von höchstens 10 Gew.-%, bezogen auf das Gewicht des Polymers, vorliegen.

18. Zusammensetzung nach einem der Ansprüche 11 bis 17, dadurch gekennzeichnet, daß das Polymer, das die starren und nicht filmbildenden Partikel bildet, ein Polymer oder Copolymer ist, das durch Polymerisation oder Copolymerisation eines Monomers oder eines Gemisches von Monomeren hergestellt ist, die unter den geradkettigen, cyclischen oder verzweigten C₁₋₁₀-Alkylacrylaten oder -methacrylaten, Styrol, Vinyltoluol, Vinylchlorid, Vinylbenzoat, Vinyl-*tert*.-butylbenzoat, Acrylsäure und Methacrylsäure ausgewählt sind.

19. Zusammensetzung nach einem der Ansprüche 11 bis 18, dadurch gekennzeichnet, daß das Polymer, das die starren und nicht filmbildenden Partikel bildet, ein vernetztes Copolymer mindestens eines geradkettigen, cyclischen oder verzweigten C₁₋₈-Alkylmethacrylats und Acrylsäure und/oder Methacrylsäure ist.

20. Zusammensetzung nach einem der Ansprüche 1 bis 19, dadurch gekennzeichnet, daß das kosmetisch akzeptable wässerige Medium aus Wasser oder einem Gemisch von Wasser und mindestens einem kosmetisch akzeptablen, mit den starren und nicht filmbildenden Partikeln und dem filmbildenden Oligomer kompatiblen Lösungsmittel ist.

21. Zusammensetzung nach Anspruch 20, dadurch gekennzeichnet, daß das Lösungsmittel unter Monoalkoholen, Polyalkoholen, Glykolethern oder Fettsäureestern oder deren Gemischen ausgewählt ist.

22. Zusammensetzung nach einem der Ansprüche 1 bis 21, dadurch gekennzeichnet, daß das kosmetisch akzeptable wässerige Medium aus Wasser oder Wasser und einem niederen C₁₋₄-Alkohol besteht.

23. Zusammensetzung nach einem der Ansprüche 1 bis 22, dadurch gekennzeichnet, daß die Konzentration an organischem Lösungsmittel im Bereich von 15 bis 35 Gew.-%, bezogen auf das Gesamtgewicht der Zusammensetzung, liegt.

24. Zusammensetzung nach einem der Ansprüche 1 bis 23, dadurch gekennzeichnet, daß sie in einer Aerosolvorrichtung unter Druck oder einem Pumpflakon konfektioniert ist.

25. Zusammensetzung nach Anspruch 24, dadurch gekennzeichnet, daß sie in einer Aerosolvorrichtung unter Druck in Gegenwart eines Treibmittels konfektioniert ist.

26. Zusammensetzung nach Anspruch 25, wobei das Treibmittel unter den flüchtigen Kohlenwasserstoffen, chlorierten und/oder fluorierten Kohlenwasserstoffen und deren Gemischen; Kohlendioxid, Distickstoffoxid, Dimethylether, Stickstoff oder Druckluft ausgewählt ist.

27. Zusammensetzung nach Anspruch 25, wobei das Treibmittel Dimethylether ist.

28. Zusammensetzung nach einem der Ansprüche 25 bis 27, wobei die Konzentration an Dimethylether im Bereich von 30 bis 55 Gew.-%, bezogen auf das Gesamtgewicht der Zusammensetzung, liegt.

29. Zusammensetzung nach einem der Ansprüche 24 bis 28, wobei die Konzentration an flüchtiger organischer Verbindung (FOV) höchstens 55 Gew.-%, bezogen auf das Gesamtgewicht der in einer Spraydose oder einem Pumpflakon konfektionierten Zusammensetzung, liegt.

30. Zusammensetzung nach einem der Ansprüche 1 bis 29, dadurch gekennzeichnet, daß in dem Gemisch, das aus den starren und nicht filmbildenden Partikeln und dem filmbildenden Oligomer besteht, die Mengenanteile der Partikel im Bereich von 5 bis 95 Gew.-% Trockensubstanz, bezogen auf das Gewicht des Gemisches, liegen.

31. Zusammensetzung nach einem der Ansprüche 24 bis 30, dadurch gekennzeichnet, daß in dem Gemisch, das aus den starren und nicht filmbildenden Partikeln und dem filmbildenden Oligomer besteht, die Anteile an Partikeln im Bereich von 40 bis 95 Gew.-% Trockensubstanz, bezogen auf das Gewicht des Gemisches, liegen.

32. Zusammensetzung nach einem der Ansprüche 24 bis 30, dadurch gekennzeichnet, daß in dem Gemisch, das aus den starren und nicht filmbildenden Teilchen und dem filmbildenden Oligomer besteht, die Anteile an Partikeln im Bereich von 70 bis 95 Gew.-% Trockensubstanz, bezogen auf das Gewicht des Gemisches, liegen.

33. Zusammensetzung nach einem der Ansprüche 1 bis 32, dadurch gekennzeichnet, daß sie ferner einen Weichmacher enthält.

34. Verwendung der Kombination, die aus den starren und nicht filmbildenden Partikeln und dem filmbildenden Oligomer nach einem der Ansprüche 1 bis 33 besteht, als Mittel zum Überziehen von Keratinmaterialien bei der Herstellung oder zur Herstellung einer kosmetischen Zusammensetzung oder zur Herstellung einer dermatologischen Zusammensetzung.

35. Produkt für das Haar zur Formgebung und/oder den Halt der Frisur, dadurch gekennzeichnet, daß es aus einer Zusammensetzung nach einem der Ansprüche 1 bis 33 besteht.

36. Produkt für das Haar nach Anspruch 35, dadurch gekennzeichnet, daß es das Gemisch, das aus den starren und nicht filmbildenden Partikeln und dem filmbildenden Oligomer besteht, in Mengenanteilen im Bereich von 3 bis 20 Gew.-% Trockensubstanz, bezogen auf das Gesamtgewicht der Zusammensetzung, enthält.

37. Produkt für das Haar nach Anspruch 35 oder 36, dadurch gekennzeichnet, daß es sich um ein Produkt handelt, das unter den Aerosollacken oder Pumpflakonlacken zur Fixierung der Haare, Lotionen für Wasserwellen, Lotionen für Fönfrisuren und Frisierschäumen ausgewählt ist.

38. Produkt zum Überziehen der Wimpern und Augenbrauen, dadurch gekennzeichnet, daß es aus einer Zusammensetzung nach einem der Ansprüche 1 bis 23, 30 und 33 besteht.

39. Produkt zum Überziehen der Wimpern und Augenbrauen nach Anspruch 38, dadurch gekennzeichnet, daß es das Gemisch, das aus den starren und nicht filmbildenden Partikeln und dem filmbildenden Oligomer besteht, in Mengenanteilen im Bereich von 2 bis 15 Gew.-% Trockensubstanz, bezogen auf das Gesamtgewicht der Zusammensetzung, enthält.

40. Produkt zum Überziehen der Wimpern und Augenbrauen nach Anspruch 38 oder 39, dadurch gekennzeichnet, daß es sich um Mascara handelt.

41. Produkt zum Überziehen der Nägel, dadurch gekennzeichnet, daß es aus einer Zusammensetzung nach einem der Ansprüche 1 bis 23, 30 und 33 besteht.

42. Produkt zum Überziehen der Nägel nach Anspruch 41, dadurch gekennzeichnet, daß es das Gemisch, das aus den starren und nicht filmbildenden Partikeln und dem filmbildenden Oligomer besteht, in Mengenanteilen im Bereich von 15 bis 30 Gew.-% Trockensubstanz, bezogen auf das Gesamtgewicht der Zusammensetzung, enthält.

43. Produkt zum Überziehen der Nägel nach Anspruch 41 oder 42, dadurch gekennzeichnet, daß es unter den Nagellakken, die leicht durch Waschen entfernt werden können, Unterlacken für Nagellacke und Grundmassen zur Pflege der Nägel, die Schutzmittel, Härter und/oder Mittel zur Pflege der Nägel enthalten, ausgewählt ist.

44. Verfahren zur kosmetischen Behandlung von Keratinmaterialien, wie dem Haar, den Wimpern, den Augenbrauen oder den Nägeln, dadurch gekennzeichnet, daß es darin besteht, direkt auf die Keratinmaterialien eine kosmetische Zusammensetzung nach einem der Ansprüche 1 bis 33 aufzutragen.
